Europäisches Patentamt

European Patent Office

Office européen des brevets

(19)

(11) Publication number: **0 234 061**
**A1**

(12) **EUROPEAN PATENT APPLICATION**

(21) Application number: **86202278.7**

(22) Date of filing: **16.12.86**

(51) Int. Cl.³: **A 61 F 13/20**
**A 61 F 11/00**

(30) Priority: **14.01.86 BE 216135**

(43) Date of publication of application:
**02.09.87 Bulletin 87/36**

(84) Designated Contracting States:
**AT CH DE ES FR GB GR IT LI LU NL SE**

(71) Applicant: **Vandenbergh, Walter**
**Bremboslei, 56**
**B-2070 Ekeren(BE)**

(72) Inventor: **Vandenbergh, Walter**
**Bremboslei, 56**
**B-2070 Ekeren(BE)**

(74) Representative: **Pieraerts, Jacques et al,**
**Bureau Gevers S.A. rue de Livourne 7, Bte. 1**
**B-1050 Bruxelles(BE)**

(54) **Implement for cleaning the outer auditory duct.**

(57) The implement is formed by a shank (1) which ends in a head (2) which is provided in cross-section, with at least one but preferably a plurality of projecting ribs (3), and which has as considered in cross-section, a serrated profile the projecting ends of which are radiused.

Fig.1.

EP 0 234 061 A1

0234061

This invention relates to an implement for cleaning the outer auditory duct.

There already are various means therefor which are not always satisfactory on the one hand because they might damage or irritate the auditory duct or the eardrum, and on the other hand because they do not always insure the required cleaning action.

The middle ear is more or less coated depending on the individuals, with ear wax (cerumen), which is released by ear wax glands.

For sanitary reasons, said ear wax should be removed regularly. Besides all kinds of objects unsuitable therefor, use is generally made of small cotton sticks. Such largely-used small sticks have for disadvantage on the one hand to make possible but a rather superficial cleaning of the auditory duct wall. As the cleaning is but superficial, such small cotton sticks are then also pressed with too much force against the eardrum wall, which may cause the above-mentioned damage or irritation.

There is disclosed in U-S Patent 1,450,612, an ear-cleaner which comprises on the one side a more or less pear-shaped component with very fine lengthwise fins. Said fins are relatively superficial and the pear-shaped component which is used as cleaner, is too point-shaped to insure efficient removing of cerumen. One might indeed actually expect that said cerumen will be displaced more deeply in the ear, rather than being removed therefrom.

In an European Application 85 400403, there has been proposed an ear-cleaner which does indeed have deeper ribs, but which is in the shape of a truncated cone. One discovers surprisingly that the Inventor proposes locating the truncated cone larger base in such a way on the ear-cleaner, that one might actually expect part of that cerumen it is desired to remove, to be pushed back towards the ear-drum. The sharp edges the ear-cleaner has on all sides, are also contrary to the most elementary dispositions as regards protecting the auditory duct and eardrum.

The invention has now for object to design an implement which insures due to the structure thereof, a thorough and secure cleaning of the auditory duct, and which does not have the drawbacks on the one hand, and the dangers on the other hand of the above-described ear-cleaner.

To make such objects possible according to the invention, the implement is formed by a shank ending in a head which has in cross-section, at least one but preferably a plurality of projecting ribs, and which has in cross-section, a serrated profile the projecting ends of which are radiused.

In a preferred embodiment, said head has a pear-shaped profile in lengthwise section.

In a particularly advantageous embodiment of the invention, at least said head is comprised of a resilient material, such as rubber or a technically-equivalent material.

Other details and features of the invention will stand out from the following description, given by way of non limitative example and with reference to the accompanying drawings, in which:

Figure 1 is a front view on an enlarged scale, of the implement according to the invention, before assembling the components thereof.

Figure 2 is a section view along line II-II in figure 1.

The implement as shown in said figures is comprised of a shank 1 and a head 2. Both said components might also be comprised of a single unit.

The head 2 is that component which is designed to clean the auditory duct wall in the middle ear. The head has along a lengthwise section, a pear-shaped profile as such a shape is well fitted to the auditory duct cavity adjacent to the eardrum.

Any other lengthwise profile might naturally also be considered. A series projecting ribs 3 are present along the length and on the surface of said head 2. As considered in a cross-section, said head 2 has a serrated profile. Each said projecting ribs 3 has relative to the head proper, a height which decreases towards the head end, and the rib end is radiused.

Said head 2 at least is made from a flexible material, for example rubber. Any technically-equivalent material may be used therefor.

According to the embodiment shown by way of example, eight projecting ribs 3 are provided in a star-like arrangement. A suitable result may basically already be obtained with a single rib, but it is clear that the embodiment as shown in the figures illustrates the most natural solution.

The head 2 may be joined to shank 1 simply by pressing the ribbed portion 1' from shank 1 into the cylinder-shaped recess 4 of head 2. It is clear that both components might be manufactured as a single part.

Due to the flexibility of that material the head 2 is made of and to the ribs 3 running along the lengthwise direction over the head surface, the implement according to the invention will fit without any difficulty to the auditory duct circumference. By rotating said implement, the ribs 3 exert a scouring action which has no danger for the auditory duct walls. Due to such action, the implement has to be pressed with less force against the wall, while however better results are being noticed.

Due to the radiusing of the tops of said projecting ribs 3, the danger of causing injuries or wounds is further markedly lowered.

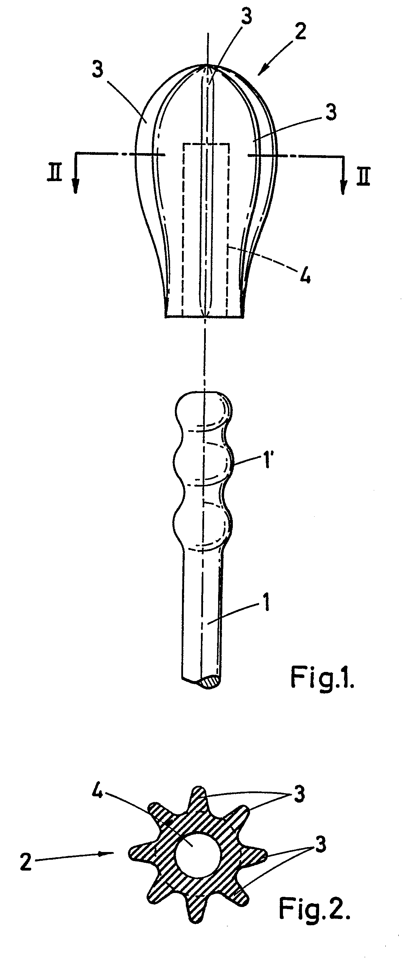

It must be understood that the invention is not limited to the above embodiments and that many changes may be brought therein without departing from the scope of the invention. For instance, the presence of eight ribs 3 may be reduced to four, two and even one for example, even if it is clear that the cross-section as shown in figure 2 insures a more regular occupation of the auditory duct and thus better guiding of the implement inside the middle ear.

0234061

CLAIMS

1. Implement for cleaning the outer auditory duct, characterized in that it is formed by a shank (1) which ends in a head (2) which is provided in cross-section, with at least one but preferably a plurality of projecting ribs (3), and has as considered in cross-section, a serrated profile the projecting ends of which are radiused.

2. Implement as defined in claim 1, characterized in that said head (2) has as considered in lengthwise section, a pear-shaped profile.

3. Implement as defined in either one of claims 1 and 2, characterized in that said head (2) at least is made from a flexible material.

4. Implement as defined in claim 3, characterized in that said flexible material is rubber or a technically-equivalent material.

5. Implement as defined in any one of claims 1 to 4, characterized in that said head (2) and shank (1) are detachable.

6. Implement as defined in claim 5, characterized in that said shank (1) has a ribbed surface (1') to enhance the securing in said head (2).

Fig.1.

Fig.2.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-1 450 612 (SCHULZ)<br>* Page 1, lines 29-70; figures * | 1-6 | A 61 F 13/38 |
| Y | | 7 | |
| Y | US-A-2 842 790 (CASTELLI)<br>* Figures 3-8; column 2, lines 1,2 * | 7 | |
| D,X | EP-A-0 158 543 (COLLIN)<br>* Claim 1; figures * | 1,2,4 | |

|  |
|---|
| **TECHNICAL FIELDS SEARCHED (Int. Cl.4)**<br><br>A 61 F<br>A 45 D |

The present search report has been drawn up for all claims

| Place of search | Date of completion of the search | Examiner |
|---|---|---|
| THE HAGUE | 03-04-1987 | STEENBAKKER J. |